# EUROPÄISCHE PATENTSCHRIFT

(11) **EP 0 352 500 B1**
(45) Veröffentlichungstag und Bekanntmachung des Hinweises auf die Patenterteilung: **08.09.1993**
(21) Anmeldenummer: 89112014.9
(22) Anmeldetag: 01.07.1989
(51) Int. Cl.: A61K 39/395, A61K 35/16

(54) **Verfahren zur Herstellung eines intravenös verabreichbaren polyklonalen immunoglobulin-Präparates mit hohemIgM-Gehalt.**
Method for the production of a polyclonal immunoglobulin-based intravenous preparation with a high IgM content .
Procédé pour la production d une preparation à base d une immuno globuline polyclonale par voie intraveneuse avec un contenu éléve en IgM.

(30) Priorität: 27.07.1988 DE 3825429
(43) Veröffentlichungstag der Anmeldung: 31.01.1990
(73) Patentinhaber: BIOTEST PHARMA GMBH, D-63303 Dreieich (DE)
(72) Erfinder: Möller, Wolfgang, Dr., D-6370 Oberursel (DE); Dichtelmüller, Herbert, Dr., D-6231 Sulzbach/Ts. (DE); Kothe, Norbert, Dr., D-6242 Kronberg (DE); Rudnick, Dieter, Dr., D-6074 Rödermark (DE); Piechaczek, Detlef, Dr., D-6115 Münster (DE)
(74) Vertreter: Wolff, Hans Joachim, Dr.jur. Dipl.-Chem.

(56) Entgegenhaltungen:
- EP-A- 162 462
- EP-A- 0 123 029
- EP-A- 0 303 088
- EP-A- 0 345 543
- EP-B- 0 013 901
- US-A- 4 371 520
- VOX SANG., vol. 52, 1987; pp. 281-290#
- CHEMICAL ABSTRACTS, vol. 107, Columbus, OH (US); no. 37654v#
- CHEMICAL ABSTRACTS, vol. 106, Columbus, OH (US); no. 117691z#
- CHEMICAL ABSTRACTS, vol. 105, Columbus, OH (US); no. 170135b#
- PATENT ABSTRACTS OF JAPAN, no. 9 (41)(c267); 21 February 1985#
- JOURNAL IMMUNOL. METHODS, vol. 91, 1986; pp. 231-235#

## Beschreibung

Immunglobuline spielen bei der Infektabwehr beim Menschen eine sehr wichtige Rolle. Die Immunglobuline sind dabei nicht einheitlich, sondern lassen sich in verschiedene Klassen mit verschiedenen biochemischen und physiologischen Eigenschaften aufteilen. Bei der Abwehr gegen virale Erreger sind besonders die IgG-Moleküle beteiligt, während die IgM-Antikörper vorzugsweise bakterielle Infektionen bekämpfen.

IgM ist aufgrund seiner pentameren Struktur besonders gut zur Agglutinierung von Bakterien geeignet. Ausserdem aktiviert IgM 100 - 400 mal mehr Komplement als IgG und ist bei der Opsonierung von Bakterien 100 mal wirksamer als das monomere IgG.

Die Gabe von IgM-haltigen Präparaten sollte deshalb zur Therapie von bakteriellen Infektionen besonders geeignet sein. Immunglobulinpräparate werden seit über 30 Jahren in der Klinik zur Therapie und Prophylaxe verschiedenster Krankheiten erfolgreich eingesetzt. Es handelt sich dabei aber überwiegend um reine IgG-Präparationen, eventuell mit Spuren an IgA und Igm. Waren die ersten Präparate nur intramuskulär verträglich, so stehen seit über 20 Jahren auch intravenös applizierbare IgG-Präparate zur Verfügung. Die Verfahrensschritte, die zur Senkung der antikomplementären Aktivität und damit zur intravenösen Verträglichkeit führen, sind in der Literatur (7 - 10) beschrieben.

Sämtliche Verfahren waren bis 1980 nur auf IgG angewendet worden. In EP 13 901 wurde dann 1980 zum ersten und bisher einzigen Mal ein intravenös verträgliches IgM-Präparat (Pentaglobin^{(R)}) beschrieben. Dieses nach Behandlung mit 0,05 bis 0,15% β-Propiolacton intravenös verträgliche Immunglobulinpräparat enthält neben 10% IgM noch 80% IgG und 10% IgA.

Andere Immunglobulinpräparate, wie sie z.B. in den Patenten: SU-PS 836 831 oder DE-PS 2 404 265 beschrieben werden, sind in ihrem IgM-Anteil mit 20 - 30% höher angereichert, dafür aber nicht intravenös verträglich.

Auch immunologisch reine IgM-Präparate, wie von Van der Hofen, Immunochemistry 1973, 10, 107-114, beschrieben, eignen sich wegen ihrer hohen antikomplementären Aktivität nicht zur intravenösen Anwendung und stehen deshalb bisher nicht für eine Therapie bakterieller Infektionen zur Verfügung.

Die einzige Möglichkeit der Applikation von IgM-Präparaten mit einem IgM-Anteil im Bereich von mehr 20% bestand bisher nur in der intramuskulären Gabe. Abgesehen davon, dass diese Applikation sehr schmerzhaft ist, lassen sich auf diese Weise keine grösseren Mengen IgM verabreichen und die Konzentration des Igm im Blut lässt sich so nicht nennenswert erhöhen.

In der älteren nachveröffentlichten EP-A 0 345 543 werden IgM-Konzentrate beschrieben, die mittels Oktansäurefällung und anschließender Anionenaustauschchromatographie hergestellt werden. Angaben über die antikomplementäre Aktivität sind jedoch nicht gemacht.

In der älteren, nachveröffentlichten EP-A 0 303 088 wird ein gereinigtes IgM-Präparat offenbart, welches aus einer Quelle monoklonaler Antikörper und mittels eines vielstufigen, sorgfältig kontrollierten Reinigungsverfahrens hergestellt wird. Aussagen über polyklonale IgM-Produkte oder eine Senkung der antikomplementären Aktivität werden jedoch nicht getroffen.

Das Ziel vorliegender Erfindung war die Bereitstellung eines hochgereinigten, zur intravenösen Anwendung geeigneten IgM-Konzentrates für die Therapie und Prophylaxe bakterieller Infektionen.

Dieses Ziel wird durch ein Verfahren zur Herstellung eines Immunglobulin-Präparates erreicht, das mindestens 50 Gew.-% IgM, bezogen auf den Gesamtimmunglobulingehalt, enthält, eine niedrige antikomplementäre Aktivität von höchstens 25 µl Komplement (1:30)/mg Protein aufweist, in wässriger Lösung stabil und frei von Viren ist. Ein solches Präparat kann aus der aus Plasma oder anderen Quellen menschlichen oder tierischen Ursprungs gewonnenen IgM-haltigen Fraktion durch Behandlung mit einem Ionenaustauscher, Eluieren des Austauschers mit einem Salz- oder pH-Gradienten und Gelfiltration hergestellt werden, wobei vor oder nach der Chromatographie mit β-Propiolacton behandelt und mit PEG 4000 in einer Konzentration von etwa 1-3% gefällt und gegebenenfalls erhitzt wird. Daran anschliessen können sich gegebenenfalls an sich bekannte Massnahmen, die auch der Sterilisierung dienen, wie Behandlung mit β-Propiolacton und UV-Licht oder Behandlung mit Solventien und Detergentien oder Pasteurisieren.

Vorzugsweise beträgt die Konzentration an IgM mindestens 50%. Das injizierbare Präparat ist eine Lösung,in der das erfindungsgemässe Präparat in einer Konzentration von 1 bis 20, vorzugsweise 3-5 g/100 ml, vorliegt.

Überraschenderweise wurde gefunden, dass das nach dem erfindungsgemässen Verfahren hergestellte IgM-Präparat mit einem IgM-Anteil von mehr als 50% in seiner antikomplementären Aktivität so weit gesenkt war, dass es intravenös verträglich war. Dieses Ergebnis war selbst nach den in EP 13 901 dargestellten Ergebnissen nicht zu erwarten, da es sich dort nur um eine rund 10%ige IgM-Lösung handelt, die zudem noch durch einen Anteil von 90% IgG und IgA stabilisiert wird.

Die antibakterielle Wirksamkeit des erfindungsgemässen Präparats wurde in Tierexperimenten im Vergleich zu dem 10%igen IgM-Präparat aus EP 13 901 überprüft. Überraschenderweise wurde dabei gefunden, dass die Wirksamkeit des erfindungsgemässen Präparats höher war, als dies nach dem IgM-Gehalt zu erwarten war. Völlig unerwartet wurde gefunden, dass sich die antibakterielle Wirksamkeit des IgM-Konzentrats dadurch erhöhen liess, dass man den Gehalt an IgG und IgA absenkte. Dies bedeutet mit anderen Worten, dass gleiche Konzentrationen an IgM dann besonders wirksam sind, wenn möglichst wenig IgG und IgA vorhanden sind.

Mit den bisher nach dem Stand der Technik erhältlichen IgM-Präparaten ist ein solcher Effekt nicht zu erzielen, da entweder die intramuskulär applizierbare Menge zu gering ist oder der IgG- und IgA-Anteil zu hoch sind.

Ein erfindungsgemässes Präparat kann auf folgendem Wege hergestellt werden:
Eine IgM-haltige Fraktion, vorzugsweise die Cohn-Fraktion lli aus der Cohn'schen Alkoholfraktionierung oder die bei der chromatographischen Isolierung von IgG aus plasma anfallende IgM-Fraktion, wird mit 1 bis 5% Oktansäure, vorzugsweise 2,5% Oktansäure, gefällt. Der IgM-haltige Überstand wird anschliessend auf einen Anionenaustauscher, z.B. mit DEAE, QAE oder QMA-Gruppen bei pH 5,5-7,5 aufgetragen. Die IgM-Fraktion wird gebunden und anschliessend mit einem Salzgradienten oder einem pH-Gradienten eluiert. Nach einem Ankonzentrierungsschritt durch Ultrafiltration wird das IgM-Eluat mit 0,05 bis 5 ml β-Propiolacton pro 100 ml IgM-Lösung behandelt. Diese Reaktion wird vorzugsweise bei 20-37°C und bei pH 7,0 bis 9,0, vorzugsweise pH 8,0 für 1 bis 10 Stunden durchgeführt, bis das β-Propiolacton vollständig verbraucht worden ist. Zur weiteren Reduktion der antikomplementären Aktivität wird die IgM-Lösung mit 1 - 3 % PEG 4000, vorzugsweise 2,5 % PEG bei 0-10°C, vorzugsweise 5°C, pH 4,5 - 5 versetzt und der entstehende Niederschlag abzentrifugiert.

Bei hohen Ausgangswerten für die antikomplementäre Aktivität kann man die IgM-Lösung gegebenenfalls zusätzlich für 0,5 bis 4 Stunden, vorzugsweise 1 Stunde auf 40 - 60°C, vorzugsweise 57°C, erhitzen.

Nach dieser Behandlung beträgt die Reinheit des IgM-Konzentrates über 50 % IgM bezogen auf das Gesamtimmunglobulin. Zur weiteren Aufreinigung kann die Lösung über ein Gelchromatographiematerial mit einer Ausschlussgrenze von über 500.000 D, z.B. Sephacryl S400HR^{(R)}, S300HR^{(R)} oder Sephorose CL6B^{(R)} chromatographiert werden. Die Massnahmen zur Senkung der antikomplementären Aktivität können auch in anderer Reihenfolge durchgeführt werden. Z.B. kann man auch vor der Anionenaustauschchromatographie mit β-Propiolacton behandeln und nach der Ausschlusschromatographie erhitzen oder man erhitzt vor der Behandlung mit β-Propiolacton und der PEG-Fällung.

Die IgM-Fraktion wird gesammelt, in bekannter Weise aufgearbeitet und sterilfiltriert.

Die folgenden Beispiele erläutern das erfindungsgemässe Herstellungsverfahren weiter.

Dabei bedeuten
DEAE-Trisacryl-LS^{(R)} = Copolymer des primären Monomeren N-Acryloyl-2-amino-2-hydroxy-methyl-1,3-propanediol und eines Diethyl-aminoethylderivates des Monomers
QA-Trisacryl^{(R)} = Copolymer des primären Monomers N-Acryloyl-2-amino-2-hydroxy-methyl-1,3-propandiol und eines Trimethylaminomethylderivates des Monomers
QMA-Accell^{(R)} = amorphes Kieselgel mit einem Acrylamid-Copolymer, enthaltend quaternäre funktionelle Aminomethylgruppen
Sephacyl S300 HR^{(R)} = quervernetztes Copolymer von Allyldextran und N,N'-Methylen-bis-acrylamid mit einem geeigneten Ausschlußbereich für globuläre Proteine mit einem MW von 1x10⁴ - 1,5x10⁶
Sephacryl S 400 HR^{(R)} = quervernetztes Copolymer von Allyldextran und N,N'-Methylen-bis-acrylamid mit einem geeigneten Ausschlußbereich für globuläre Proteine mit einem MW von 2x10⁴-8x10⁶
Tween/Tween 80^{(R)} = Polyoxyethylensorbitan-monooleat.

### Beispiel 1

1 kg Cohn-Paste III wurde in 5 l 0,1 M Acetatpuffer, pH 5, gelöst und mit 2,5% Oktansäure bei 25°C versetzt. Nach 4 Stunden wurde der Niederschlag abzentrifugiert und der Überstand gegen 0,025M Trishydroxymethylaminomethan, pH 6,5, diafiltriert. Die Lösung wurde dann auf eine 3 l Säule mit QA-Trisacryl-LS^{(R)} in gleichem Puffer aufgetragen. Das IgM wurde an den Träger gebunden und anschliessend mit 0,3 M NaCl eluiert. Das Eluat wurde auf einen Proteingehalt von 40 g/l ankonzentriert, 1 h auf 57°C erhitzt und mit 0,15% β-Propiolacton über Nacht bei 25°C und pH 8,0 behandelt. Die Lösung wurde anschliessend mit 2,5 g pro 100 ml PEG 4000 bei pH 4,5 versetzt, 1 Stunde bei 4°C gerührt und zentrifugiert. Der Überstand wurde dann über eine 20 l Säule mit Sephacryl S400 HR^{(R)} chromatographiert. Die 2. Fraktion, die das IgM enthielt, wurde ultrafiltriert und sterilfiltriert. Der IgM-Gehalt betrug 85% bei 5% IgG und 9% IgA. Der Gesamtimmunglobulingehalt betrug 99%.

### Beispiel 2

Ein Pool aus 50 l Humanplasma wurde bei 4°C aufgetaut und das Kryopräzipitat abgetrennt. Nach der Entfernung der PPSB-Faktoren mit DEAE-Sephadex^{(R)} und des Fibrinogens durch Fällung mit 9% Ethanol bei pH 5,3 wurde das Restplasma auf ein Ionenmilieu von 22 mM Trishydroxymethylaminomethan/HCl, pH 7,5, eingestellt. Anschliessend wurde über eine mit gleichem Puffer äquilitierte 10 l Säule mit DEAE-Trisacryl-LS^{(R)} chromatographiert. Das gebundene IgM wurde mit 0,3 M NaCl, pH 7,0, eluiert. Das Eluat wurde mit 25% Ethanol bei -3°C und pH 7,5 gefällt und der Niederschlag in 0,1 M Acetat-Puffer, pH 5, auf einen Proteingehalt von 50 g/l gelöst. Die Lösung wurde mit 2,5% Oktansäure bei 25°C und pH 5 versetzt und der Niederschlag abgetrennt. Der Überstand wurde mit 0,11% β-Propiolacton für 5 Stunden bei 25°C und pH 7 behandelt und anschliessend mit 2,5 g/100 ml PEG 4000 für 1 Stunde bei 4°C gefällt und zentrifugiert. Anschliessend wurde der Überstand in 3 Läufen über eine 20 l Säule mit Sephacryl S400^{(R)} chromatographiert. Die 2. Fraktion wurde ultrafiltriert und sterilfiltriert. Diese 2. Fraktion enthielt das IgM in einer Reinheit von 93% bei 2% IgG und 5% IgA. Der Gesamtimmunglobulinanteil betrug 100%.

### Beispiel 3

1 kg Paste III wurde analog zu Beispiel 1 mit Oktansäure gefällt und der Überstand gegen 0,025 M Trishydroxymethylaminomethan, pH 7,0 diafiltriert. Die Lösung wurde dann auf eine 3 l Säule QMA-Accell^{(R)} in gleichem Puffer aufgetragen.
Das IgM wurde an den Träger gebunden und nach dem Waschen der Säule mit 0,05 M Natriumacetat, pH 4,5 eluiert. Das Eluat wurde analag zu Beispiel 1 mit PEG 4000 und β-Propiolacton behandelt. Anschliessend wurde der Überstand über eine 2 l Säule Sephadex G 25^{(R)} umgepuffert, ultrafiltriert und sterilfiltriert.
Der IgM-Gehalt betrug 73 % bei 20 % IgA und 7 % IgG. Der Gesamtimmunglobulin-Gehalt betrug 100 %

Zusätzlich zu der Behandlung mit β-Propiolacton kann auch eine Behandlung mit β-Propiolacton und UV-Licht oder mit Detergentien und Solventien, vorzugsweise Tri-n-butylphosphat und TWEEN 80^{(R)}, oder eine Pasteurisierung treten. Diese Behandlung kann ebenso wie das Erhitzen auch im Anschluss an die Gelfiltration erfolgen.

Gegebenenfalls können dem Präparat auch Proteine, vorzugsweise Humanalbumin, Zucker, vorzugsweise Maltose, oder Aminosäuregemische zugesetzt werden.

Ein nach dem erfindungsgemässen Verfahren hergestelltes IgM-Konzentrat wurde mit dem als Ausgangsmaterial dienenden Handelsprodukt Pentaglobin, das IgG, IgA und IgM enthält, sowie mit einer ebenfalls aus diesem Ausgangsmaterial hergestellten IgG-Fraktion verglichen. Die Ergebnisse sind nachfolgend dargestellt.

### 1. Charakterisierung der Vergleichspräparate

Die Bestimmung der Immunglobuline IgG, IgA und IgM erfolgte mit Antiseren nephelometrisch. Die Bestimmung des Gesamtproteingehaltes erfolgte mit der Biuret-Methode. Die Daten der einzelnen Prüfpräparate sind in Tabelle 1 zusammengestellt.

**Tabelle 1**

| Daten der Prüfpräparate | | | | | |
|---|---|---|---|---|---|
| Präp.-Nr. | Präparat | Prot. (g/l) | IgG | IgA | IgM |
| | | | (mg/100 ml) | | |
| 1 | Pentaglobin^{(R)} | 51,5 | 3720 | 920 | 750 |
| 2 | IgG-Fraktion | 48,9 | 3770 | 890 | 70 |
| 3 | IgM-Konzentrat | 8,1 | 40 | 70 | 750 |

### 2. Tierexperimentelle Prüfung

Die antibakterielle Wirksamkeit des erfindungsgemässen IgM-Konzentrates (Präp. Nr. 3) wurde im Vergleich zu Pentaglobin (Nr. 1) und einer IgG-Fraktion (Nr. 2) in der Pseudomonas-infizierten Maus durchgeführt (1). Die Überlebensraten sind in Tabelle 2 zusammengestellt.

**Tabelle 2**

| Ergebnisse des Mäuseschutz-Tests | | |
|---|---|---|
| Präp.-Nr. | Präparat | geschützte Mäuse (%) 21 Stunden nach Infektion |
| 1 | Pentaglobin^{(R)} | 47,6 |
| 2 | IgG-Fraktion | 33,3 |
| 3 | IgM-Konzentrat (erfindungsgemäss) | 66,7 |
| 4 | unbehandelt | 9,5 |

Das erfindungsgemässe IgM-Konzentrat (3) hat demnach bei einem Proteingehalt von 1/5 der Präparate 1 und 2 eine signifikant höhere Schutzwirkung als diese Präparate.

### 3. Bestimmung der antikomplementären Aktivität (ACA)

Die ACA - als Mass für die intravenöse Verträglichkeitwurde nach der Methode von Kabat und Mayer (2) bestimmt. Die Ergebnisse für handelsübliche Präparate im Vergleich zum erfindungsgemässen IgM-Konzentrat - jeweils als 5%ige Lösungen getestet - sind in Tabelle 3 zusammengestellt.

**Tabelle 3**

| ACA von i.v. Immunglobulinpräparaten | | | |
|---|---|---|---|
| Nr. | Prüfpräparat | IgM (%) | ACA (µlC 1:30/mg Prot.) |
| 1 | Intraglobin^{(R)} | 0 | 10 |
| 2 | Pentaglobin^{(R)} | 10 | 25 |
| 3 | erfindungsgemässes IgM-Konzentrat | 75 | 25 |

Der Wert für das erfindungsgemässe IgM-Konzentrat ist vergleichbar mit dem seit 1985 verfügbaren IgM-haltigen Präparat Pentaglobin^{(R)}.

### 4. Versuche zur Virus-Inaktivierung

Das erfindungsgemässe IgM-Konzentrat wurde mit Bakteriophagen vom Typ Φ x 174 und mit Sendai-Viren versetzt. Sterilisiert wurde mit β-Propiolacton (3), β-PL/UV (4), Solvent/Detergent (3) und durch Pasteurisation (10 h, 60°C) (5). Tabelle 4 gibt die Versuchsergebnisse wieder.

**Tabelle 4**

| Virusinaktivierung in IgM-Konzentraten | | | |
|---|---|---|---|
| Protein-Konz. (g/100 ml) | Virus Typ | Sterilisationsmethoden | Inaktivierung (log₁₀+) |
| 4 | Φ x 174 | β-PL | >7 |
| 4 | Φ x 174 | β-PL/UV | 7 |
| 0,5 | Sendai | Solv/Det. | >4,5 |
| 5 | Φ x 174 | past. | >8,0 |

Die Ergebnisse zeigen eine so hohe Sterilisationseffektivität der einzelnen Verfahren, dass eine Virusübertragung eines nach einem der Verfahren der Tabelle 4 sterilisierten IgM-Konzentrates auszuschliessen ist.

### 5. Versuche zur Lagerstabilität

Das erfindungsgemässe IgM-Konzentrat wurde als 1,6%ige Lösung (1,2 g/100 ml Igm) 4 h auf 57°C erhitzt. Es wurde mit der passiven Hämagglutinationsmethode (PHA) nach Neter (6) auf Antikörper gegen folgende Bakterien geprüft: E. coli, Klebsiellen, Streptokokken. Die Aktivitäten vor bzw. nach der Erhitzung sind in Tabelle 5 zusammengestellt.

**Tabelle 5**

| Reziproke antibakterielle Ak-Titer in IgM-Konzentraten im Vergleich zu Pentaglobin^{(R)} | | | | |
|---|---|---|---|---|
| Anti- | IgM-Konz. | Pentaglobin | IgM-Konz. | Pentaglobin |
| | vor Erhitzen | | nach Erhitzen | |
| E. coli | 640 | 160 | 320 | 160 |
| Klebsiellen | 1280 | 640 | 640 | 320 |
| Streptokokken | 320 | - | 160 | - |
| Strep.virid. | 320 | 160 | 160 | 40 |

Das erfindungsgemässe IgM-Konzentrat ist in seinen immunologischen Aktivitäten unter Berücksichtigung der Fehlerbreite der Bestimmungsmethoden (±1 Titerstufe) hitzestabil. Es verhält sich bezüglich der Lagerstabilität wie das Igm-haltige Handelspräparat Pentaglobin^{(R)}.

## Patentansprüche

1. Verfahren zur Herstellung eines intravenös verabreichbaren polyklonalen Immunglobulin-Präparates zur Therapie und Prophylaxe von bakteriellen Infektionen, enthaltend
a) mindestens 50 Gew.-% IgM, bezogen auf den vorhandenen Gesamtimmunglobulingehalt,
b) eine antikomplementäre Aktivität von höchstens 25µl Komplement (1:30)/mg Protein aufweisend, welches
c) frei von Viren ist und
d) eine Proteinkonzentration von 1 bis 20 g/100 ml aufweist,
dadurch gekennzeichnet, daß man die immunglobulinhaltige Fraktion mit einem Ionenaustauscher behandelt, diesen mit einem Salz- oder pH-Gradienten eluiert, das Eluat einer Gelfiltration unterwirft und vor oder nach der Anionenaustauschchromatographie oder der Gelfiltration mit β-Propiolacton und mit PEG 4000 in einer Konzentration von etwa 1 bis 3% behandelt und gegebenenfalls erhitzt.

2. Verfahren nach Anspruch 1, dadurch gekennzeichnet, daß man als Anionenaustauscher ein Copolymer aus dem primären Monomer N-Acryloyl-2-amino-2-hydroxymethyl-1,3-propandiol und einem Diethyl-aminoethylderivat des Monomers, ein Copolymer der primären Monomeren N-Acryloyl-2-amino-2-hydroxy-methyl-1,3-propandiol und einem Trimethylaminomethylderivat des Monomeren, oder amorphes Kieselgel mit einem Acrylamid-Copolymer, enthaltend funktionelle quaternäre Aminomethylgruppen verwendet.

3. Verfahren nach Anspruch 1 oder 2, dadurch gekennzeichnet, daß man als Gel für die Gelfiltration ein quervernetztes Copolymer von Allyldextran und N,N'-Methylen-bis-acrylamid mit einem geeigneten Ausschlußbereich für globuläre Proteine mit einem MW von 1x10⁴-1,5x10⁶, oder quervernetztes Copolymer von Allyldextran und N,N'-Methylen-bis-acrylamid mit einem geeigneten Ausschlußbereich für globuläre Proteine mit einem MW von 1x10⁴-8x10⁶ verwendet.

4. Verfahren nach einem der Ansprüche 1 bis 3, dadurch gekennzeichnet, daß man das Präparat vor oder nach der Anionenaustauschchromatographie oder der Gelfiltration mit β-Propiolacton oder β-Propiolacton und UV-Licht und mit 1-3% PEG 4000 bei 0-10°C, vorzugsweise 5°C, und pH 4,5-5 behandelt.

5. Verfahren nach einem der Ansprüche 1 bis 4, dadurch gekennzeichnet, daß man 0,5 bis 5 Stunden lang bei 40 bis 60°C, vorzugsweise 1 Stunde lang bei 57°C, erhitzt.

6. Verfahren nach einem der Ansprüche 1 bis 5, dadurch gekennzeichnet, daß man das Präparat mit Solventien und Detergentien, vorzugsweise Tri-n-butylphosphat und Polyoxyethylensorbitan-monooleat vor oder nach der Gelfiltration behandelt.

7. Verfahren nach einem der Ansprüche 1 bis 5, dadurch gekennzeichnet, daß man das Präparat vor oder nach der Gelfiltration pasteurisiert.

8. Verfahren nach einem der Ansprüche 1 bis 7, dadurch gekennzeichnet, daß man dem Präparat Proteine, vorzugsweise Humanalbumin, Zucker, vorzugsweise Maltose, oder Aminosäuregemische zusetzt.

9. Verfahren nach einem der Ansprüche 1 bis 8, dadurch gekennzeichnet, daß man das Präparat als Lösung mit einer Proteinkonzentration von 3-5 g/100 ml herstellt.

10. Verfahren nach einem der Ansprüche 1 bis 9, dadurch gekennzeichnet, daß man als Ausgangsmaterial immunglobulinhaltige Plasmafraktionen menschlichen oder tierischen Ursprungs verwendet.

## Claims

1. A process for the preparation of an intravenously administrable polyclonal immunoglobulin preparation for the therapy and prophylaxis of bacterial infections, containing
a) at least 50% by weight of IgM, based on the total immunoglobulin content present and
b) an anticomplementary activity having at most 25 µl of complement (1:30)/mg of protein, which
c) is free from viruses and
d) has a protein concentration of from 1 to 20 g/100 ml,
characterised in that the immunoglobulin-containing fraction is treated with an ion exchanger, the latter is eluted with a salt gradient or a pH gradient, the eluate is subjected to a gel filtration and treated either before or after the anion exchange chromatography or gel filtration with β-propiolactone and with PEG 4000 at a concentration of about 1 to 3% and optionally heated.

2. A process according to Claim 1, characterised in that the anion exchanger used is a copolymer of the primary monomer N-acryloyl-2-amino-2-hydroxy-methyl-1,3-propanediol and a diethyl-aminoethyl derivative of the monomer, a copolymer of the primary monomer N-acryloyl-2-amino-2-hydroxy-methyl-1,3-propanediol and a trimethylaminomethyl derivative of the monomer or amorphous silica gel with an acrylamide copolymer containing functional quaternary aminomethyl groups.

3. A process according to Claim 1 or 2, characterised in that the gel used for gel filtration is a transversely cross-linked copolymer of allyl dextran and N,N'-methylene-bis-acrylamide having a suitable exclusion range for globular proteins with an MW of from 1x10⁴-1.5x10⁶ or a transversely cross-linked copolymer of allyl dextran and N,N'-methylene-bis-acrylamide having a suitable exclusion range for globular proteins with an MW of from 1x10⁴-8x10⁶.

4. A process according to one of the Claims 1 to 3, characterised in that the preparation is treated either before or after the anion exchange chromatography or gel filtration with β-propiolactone or β-propiolactone and UV light and with 1 to 3% of PEG 4000 at 0-10°C, preferably at 5°C, and at pH from 4.5 to 5.

5. A process according to one of the Claims 1 to 4, characterised in that heating is carried out for from 0.5 to 5 hours at from 40 to 60°C, preferably for 1 hour at 57°C.

6. A process according to one of the Claims 1 to 5, characterised in that the preparation is treated with solvents and detergents, preferably tri-n-butylphosphate and polyoxyethylene sorbitan mono-oleate, before or after gel filtration.

7. A process according to one of the Claims 1 to 5, characterised in that the preparation is pasteurised before or after gel filtration.

8. A process according to one of the Claims 1 to 7, characterised in that proteins, preferably human albumin, sugar, preferably maltose, or amino acid mixtures are added to the preparation.

9. A process according to one of the Claims 1 to 8, characterised in that the preparation is prepared as a solution having a protein concentration of from 3 to 5 g/100 ml.

10. A process according to one of the Claims 1 to 9, characterised in that the starting material used is immunoglobulin-containing plasma fractions of human or animal origin.

## Revendications

1. Procédé de production d'une préparation d'immunoglobulines polyclonales administrable par voie intraveineuse, pour le traitement et la prévention d'infections bactériennes,
a) contenant au moins 50 % en poids d'IgM, par rapport à la teneur totale en immunoglobulines présente,
b) présentant une activité anticomplémentaire atteignant au maximum 25 µl de complément (1:30)/mg de protéine,
c) exempte de virus et
d) présentant une concentration en protéines de 1 à 20 g/100 ml, caractérisé en ce qu'on traite la fraction contenant des immunoglobulines à l'aide d'un échangeur d'anions, qu'on élue ce dernier à l'aide d'un gradient de sel ou de pH, qu'on soumet l'éluat à une filtration sur gel et qu'on traite, avant ou après la chromatographie par échange d'anions ou la filtration sur gel, à l'aide de β-propiolactone et de PEG 4000 à une concentration d'environ 1 à 3 % et qu'on chauffe le cas échéant.

2. Procédé selon la revendication 1, caractérisé en ce qu'on utilise comme échangeur d'anions un copolymère composé du monomère primaire N-acryloyl-2-amino-2-hydroxyméthyl-1,3-propanediol et d'un dérivé diéthyl-aminoéthyle du monomère, un copolymère composé du monomère primaire N-acryloyl-2-amino-2-hydroxyméthyl-1,3-propanediol et d'un dérivé diméthylaminométhyle du monomère ou un gel de silice amorphe comportant un copolymère d'acrylamide contenant des groupements aminométhyle quaternaires fonctionnels.

3. Procédé selon la revendication 1 ou 2, caractérisé en ce qu'on utilise comme gel pour la filtration sur gel un copolymère réticulé d'allyldextrane et de N,N'-méthylène-bis-acrylamide ayant une plage d'exclusion appropriée pour des protéines globulaires ayant un poids moléculaire compris entre 1 x 10⁴ et 1,5 x 10⁶ ou un copolymère réticulé d'allyldextrane et de N,N'-méthylène-bis-acrylamide ayant une plage d'exclusion appropriée pour des protéines globulaires ayant un poids moléculaire compris entre 1 x 10⁴ et 8 x 10⁶.

4. Procédé selon une des revendications 1 à 3, caractérisé en ce qu'on traite la préparation avant ou après la chromatographie par échange d'anions ou la filtration sur gel à l'aide de β-propiolactone ou de β-propiolactone et de lumière UV et à l'aide de 1 à 3 % de PEG 4000 entre 0 et 10°C, de préférence à 5°C, et à un pH compris entre 4,5 et 5.

5. Procédé selon une des revendications 1 à 4, caractérisé en ce qu'on chauffe pendant 0,5 à 5 h, entre 40 et 60°C, de préférence pendant 1 h à 57°C.

6. Procédé selon une des revendications 1 à 5, caractérisé en ce qu'on traite la préparation à l'aide de solvants et de détergents, de préférence à l'aide de phosphate de tri-n-butyle et de monooléate de polyoxyéthylène-sorbitanne, avant ou après la filtration sur gel.

7. Procédé selon une des revendications 1 à 5, caractérisé en ce qu'on pasteurise la préparation avant ou après la filtration sur gel.

8. Procédé selon une des revendications 1 à 7, caractérisé en ce qu'on ajoute à la préparation des protéines, de préférence de l'albumine hmaine, des oses, de préférence du maltose, ou des mélanges d'acides aminés.

9. Procédé selon une des revendications 1 à 8, caractérisé en ce qu'on produit la préparation sous forme de solution ayant une concentration en protéines comprise entre 3 et 5 g/100 ml.

10. Procédé selon une des revendications 1 à 9, caractérisé en ce qu'on utilise comme substance de départ des fractions plasmatiques contenant des immunoglobulines d'origine humaine ou animale.
